# EUROPÄISCHE PATENTSCHRIFT

(11) **EP 0 760 365 B1**
(45) Veröffentlichungstag und Bekanntmachung des Hinweises auf die Patenterteilung: **03.01.2001**
(21) Anmeldenummer: 96113175.2
(22) Anmeldetag: 16.08.1996
(51) Int. Cl.: C07C 279/22, C07C 317/32, C07C 317/50, A61K 31/155, A61K 31/165, C07C 317/44

(54) **Alkenyl-Benzoylguanidine**
Alkenyl-benzoylguanidines
Alkényl-benzoylguanidines

(30) Priorität: 24.08.1995 DE 19531138
(43) Veröffentlichungstag der Anmeldung: 05.03.1997
(73) Patentinhaber: MERCK PATENT GmbH, 64293 Darmstadt (DE)
(72) Erfinder: Gericke, Rolf, Dr., 64342 Seeheim (DE); Baumgarth, Manfred, Dr., 64297 Darmstadt (DE); Minck, Klaus-Otto, Dr., 64372 Ober-Ramstadt (DE); Beier, Norbert, Dr., 64354 Reinheim (DE)

(56) Entgegenhaltungen:
- EP-A- 0 577 024
- EP-A- 0 699 663
- WO-A-96/04241
- DE-A- 4 318 756

## Beschreibung

Die Erfindung betrifft Alkenyl-benzoylguanidin-Derivate der Formel I 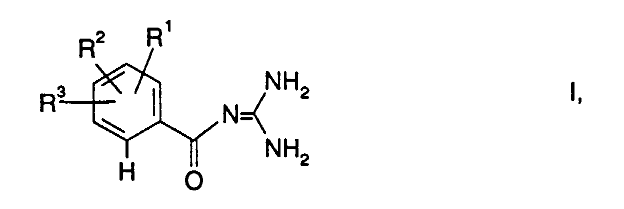 worin
- R¹ und R²: jeweils unabhängig voneinander H, Hal, A, CN, NO₂, CF₃, CH₂F, CHF₂, C₂F₅, CH₂CF₃ oder SOₙ-R⁴,
- R³: -CR⁵=CR⁶R⁷, -C(R⁶R⁵)-CR⁷=CR⁹R⁸, -C(R⁶R⁵)-C(R⁷R⁸)-CR⁹=CR¹⁰R¹¹ oder Cycloalkenyl mit 3-7 C-Atomen oder Cycloalkenylalkyl mit 4-8 C-Atomen,
- R⁴: A oder Ph,
- R⁵, R⁶, R⁷, R⁸, R⁹, R¹⁰ und R¹¹: jeweils unabhängig voneinander H oder A
- A: Alkyl mit 1 bis 6 C-Atomen,
- Hal: F, Cl, Br oder I,
- Ph: unsubstituiertes oder ein-, zwei- oder dreifach durch A, OA, NR⁴R⁵, F, Cl, Br, I oder CF₃ substituiertes Phenyl und
- n: 1 oder 2,
bedeuten,
sowie deren physiologisch unbedenkliche Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1992)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Darüber hinaus kennt man strukturell ähnliche Verbindungen beispielsweise aus EP 04 16 499.

Gegenstand der Erfindung sind Verbindungen der Formel I sowie ihre physiologisch unbedenklichen Salze.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zellproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na⁺/H⁺-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von N. Escobales and J. Figueroa in J. Membrane Biol. 120, 41-49 (1991) oder von L. Counillon, W. Scholz, H.J. Lang und J. Pouysségur in Mol. Pharmacol. 44, 1041-1045 (1993) angegeben werden.

Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe Verwendung finden.

In den angegebenen Formeln bedeutet A eine verzweigte oder unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

R¹ bedeutet vorzugsweise H, A oder Hal, insbesondere Br oder Cl. Besonders bevorzugt bedeutet R¹ Methyl oder Ethyl. Ferner aber auch vorzugsweise CH₂F, CHF₂, CF₃ oder C₂F₅. R¹ ist vorzugsweise in ortho-Position zur Guanidingruppe angeordnet.

R² bedeutet vorzugsweise A-SO₂, CF₃, Cl, Br oder NO₂. Besonders bevorzugt steht R² für H₃C-SO₂-.

Der Rest R² steht vorzugsweisein 3- oder 5-Position zur Benzoylguanidin-Gruppe.

R³ bedeutet bevorzugt -CR⁵=CR⁷R⁶ oder -C(R⁶R⁵)-CR⁷=CR⁹R⁸.

R³ bedeutet ferner auch vorzugsweise Cyclopropenyl, Cyclobutenyl, 3-Cyclopentenyl, 4-Cyclopentenyl, 3- oder 4-Cyclohexenyl, 3-, 4- oder 5-Cycloheptenyl bzw. ein entsprechendes Cycloalkenylmethyl-Derivat.

R⁴ bedeutet vorzugsweise Methyl, Ethyl oder Phenyl.

Ph bedeutet vorzugsweise unsubstituiertes oder einfach durch Cl, Br, A, OA, NH₂, NHA, NA₂ oder CF₃ substituiertes Phenyl.

R⁵ bis R¹¹ bedeuten vorzugsweise jeweils unabhängig voneinander H und/oder A, insbesondere Wasserstoff und/oder Methyl.

Hal bedeutet vorzugsweise F, Cl oder Br.

Allgemein gilt, daß sämtliche Reste wie z.B. R⁵ bis R¹¹, die mehrfach auftreten, gleich oder verschieden sein können, d.h. unabhängig voneinander sind.

Die Verbindungen können als cis-/trans-Isomere vorliegen. Die vorliegende Erfindung schließt sowohl Mischungen als auch die reinen cis- und trans-Formen der Verbindungen mit ein. Die Verbindungen können ferner auch als Racemate vorliegen, die in Enantiomere getrennt werden können.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln la bis Ih ausgedrückt werden, die der Formel I entsprechen und worin die nicht näher bezeichneten Reste die bei der Formel I angegebene Bedeutung haben, worin jedoch
- in Ia: R¹ A und R² -SO₂-A, NO₂ oder CF₃ bedeuten;
- in Ib: R¹ H oder Hal und R₂ -SO₂A, NO₂ oder CF₃ bedeuten;
- in Ic: R¹ A oder H, R²-SO₂A und R³ -CR⁵=CR⁶R⁷, mit R⁵ bis R⁷ jeweils unabhängig voneinander H oder Methyl, bedeuten;
- in Id: R¹ A oder H, R²-SO₂-A und R³ -C(R⁵R⁶)-CR⁷=CR⁸R⁹ mit R⁵ bis R⁹ jeweils unabhängig voneinander H oder Methyl, bedeuten;
- in Ie: R¹ A oder H, R² -SO₂A und R³ -C(R⁵R⁶)-CR⁷R⁸-CR⁹=CR¹⁰R¹¹, mit R⁵ bis R¹¹ jeweils unabhängig voneinander H oder Methyl, bedeuten;
- in If: R¹ H oder A bedeutet und in ortho-Position zur Benzoylguanidingruppe steht und R² -SO₂-A bedeutet und in meta-Position zur Benzoylguanidin-Gruppe steht,
- in Ig: R³ Cycloalkenyl mit 3-7 C-Atomen bedeutet und in para-Position zur Amidgruppe steht und R² in meta-Position zur Amidgruppe steht und -SO₂-A bedeutet;
- in Ih: R¹ A oder H, R²-SO²-A, R³ Cycloalkenyl mit 3 bis 7 C-Atomen bedeutet und R³ in para-Stellung zur Benzoylguanidingruppe angeordnet ist.

Gegenstand der Erfindung ist ferner ein Verfahren zur Herstellung der Verbindungen der Formel I gemäß Anspruch 1, sowie von deren Salzen, dadurch gekennzeichnet, daß man eine Verbindung der Formel II 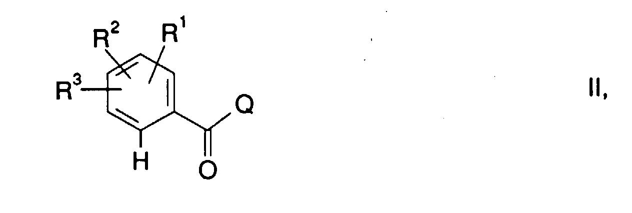 worin R¹, R² und R³ die zuvor angegebenen Bedeutungen haben
und
- Q: Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
oder daß man ein Benzoylguanidin der Formel III 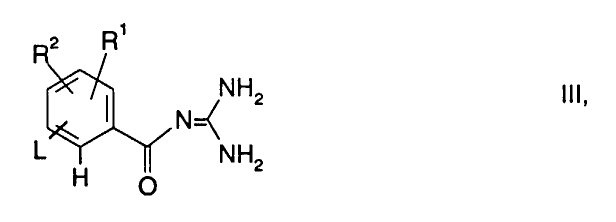 worin R¹ und R² die zuvor angegebenen Bedeutungen besitzen,
und
- L: F, Cl, Br oder I
bedeutet,
mit einer ungesättigten Kohlenwasserstoffverbindung der Formel IV

R³-H IV

worin
R³ die angegebene Bedeutung besitzt,

in Gegenwart eines Übergangsmetallkatalysators und gegebenenfalls eines Aktivators umsetzt,
oder daß man eine sonst der Formel I entsprechende Verbindung, die jedoch anstelle eines oder mehrerer Wasserstoffatome eine oder mehrere reduzierbare Gruppe(n), wie z.B. eine Alkinylgruppe und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
oder daß man einen Rest R³ in einen anderen Rest R³ durch Isomerisierung umwandelt, indem man eine Doppelbindung unter Einwirkung eines Übergangsmetallkatalysators und/oder eines Metallcarbonyls umlagert;
und/oder daß man eine erhaltene Base der Formel I durch Behandeln mit einer Säure in eines ihrer Salze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden Verbindungen der Formel I hergestellt, indem man ein aktiviertes Carbonsäurederivat der Formel II, wobei Q besonders bevorzugt Cl oder -O-CH₃ ist, mit Guanidin umsetzt. Besonders geeignet sind auch Reaktionsvarianten, bei denen die freie Carbonsäure II (Q = OH) in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol, Dicyclohexylcarbodiimid oder die Mukayama-Variante (Angew. Chem. 91, 788-812 (1979)).

Die Carbonsäuren und Carbonsäurederivate der Formel II sind in der Regel bekannt. Sie werden insbesondere durch die Heck-Reaktion, J. Org. Chem. 46, 1067 (1981) oder durch Pd-katalysierte Kreuzkupplungen hergestellt. Bevorzugte Katalysatoren sind z.B. Pd(PPh₃)₄, (Ph₃P)₂PdCl₂, Pd(CH₃COO)₂ oder Pd-(ll)-[1,1'-bis-diphenylphosphin)ferrocen]-chlorid, vorzugsweise in Gegenwart von Cul.

Die Carbonsäuren der Formel II bzw. deren Derivate werden ferner hergestellt, indem man geeignete Benzoesäurederivate der Formel V 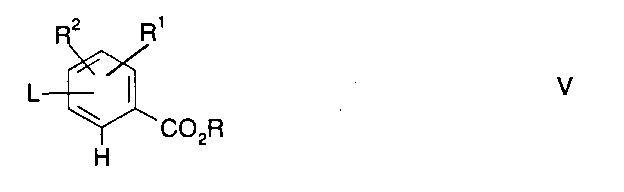 worin R¹, R² und L die angegebenen Bedeutungen besitzen und R H oder A ist,
metalliert und dann mit einem entsprechenden Alkenylhalogenid umsetzt. Eine geeignete Base zur Metallierung ist z.B. Lithiumdiisopropylamid.

Bei den zuvor genannten Kreuzkupplungen setzt man ein Carbonsäure- oder ein Esterderivat der Formel V, worin L vorzugsweise Cl, Br oder I bedeutet, mit einer metallorganischen Alkenylverbindung, die in situ durch Metallierung hergestellt wird, in Gegenwart eines geeigneten Metallkatalysators, insbesondere eines der zuvor genannten, um.

Die Umsetzung erfolgt in Analogie zu der Reaktion der Verbindungen III und IV. Sie wird nachstehend beschrieben.

Besonders bevorzugt stellt man eine Verbindung der Formel II jedoch nach der beispielsweise in J. Org. Chem. 46, 1067 (1981) beschriebenen Heck-Reaktion her, indem man beispielsweise eine Verbindung der Formel V, worin L Br oder I bedeutet, mit der cyclischen oder linearen Alkenylverbindung in Gegenwart eines Pd-ll-Salzes, z.B. des Acetates, und eines Phosphins, z.B. Tri-o-tolylphosphin, umsetzt.

Die Umsetzung eines reaktionsfähigen Carbonsäurederivates der Formel II mit Guanidin erfolgt in an sich bekannter Weise vorzugsweise in einem protischen oder aprotischen polaren oder unpolaren inerten organischen Lösungsmittel.

Geeignete Lösungsmittel werden nachfolgend für die Umsetzung der Verbindungen III und IV genannt. Besonders bevorzugte Solventien sind jedoch Methanol, THF, Dimethoxyethan, Dioxan oder daraus herstellbare Gemische sowie Wasser. Als Reaktionstemperatur sind beispielsweise Temperaturen zwischen 20° und dem Siedepunkt des Lösungsmittels geeignet. Die Reaktionszeiten liegen zwischen 5 Min. und 12 Std.. Es ist zweckmäßig, bei der Reaktion einen Säurefänger einzusetzen. Hierzu eignen sich jegliche Arten von Basen, die die Reaktion selbst nicht stören. Besonders geeignet ist jedoch die Verwendung von anorganischen Basen wie Kaliumcarbonat oder von organischen Basen wie Triethylamin oder Pyridin oder aber ein Überschuß des Guanidins.

Verbindungen der Formel I gemäß Anspruch 1 können ferner hergestellt werden, indem man ein Benzoylguanidin der Formel III mit einer Verbindung der Formel IV umsetzt. Die Ausgangsstoffe der Formel III können auf einfache Weise durch Umsetzung von entsprechend substituierten Benzoesäuren oder daraus ableitbaren reaktionsfähigen Säurederivaten, wie z.B. Säurehalogeniden, Estern oder Anhydriden, mit Guanidin, unter Reaktionsbedingungen wie sie für die Amidherstellung an sich bekannt und allgemein üblich sind, hergestellt werden. Besonders geeignet sind wiederum solche Reaktionsvarianten, wie sie zuvor für die Umsetzung von Verbindung II mit Guanidin angegeben werden.

Die Verbindungen der Formel IV sind ebenso wie die Methoden zu ihrer Herstellung an sich bekannt. Sofern sie nicht bekannt sind, können sie nach den an sich bekannten Methoden hergestellt werden.

Die Herstellung der Verbindung II sowie die Umsetzung der Verbindung III mit einer Verbindung der Formel IV erfolgt in an sich bekannter Weise, bevorzugt in einem protischen oder aprotischen polaren inerten organischen Lösungsmittel.

Bei der Herstellung von II oder bei der Umsetzung von III mit IV ist es ebenfalls zweckmäßig, in Gegenwart einer Base oder mit einem Überschuß der basischen Komponente zu arbeiten. Als Basen eignen sich bevorzugt z.B. Alkalimetall- oder Erdalkalimetallhydroxide, -carbonate, -alkoholate oder organische Basen wie Triethylamin oder Pyridin, die auch im Überschuß angewendet werden und dann gleichzeitig als Lösungsmittel dienen können.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Die Heck-Reaktion erfordert gewöhnlich erhöhte Temperaturen (100-150°C).

Eine besonders bevorzugte Arbeitsweise bei der Umsetzung von III mit IV besteht darin, daß man das entsprechende Benzoylguanidin und die ungesättigte Kohlenwasserstoffverbindung zusammen mit einem Pd(ll)-Katalysator und einem Triphenylphosphinderivat in DMF löst und dann erhitzt. Ist das Alken gasförmig, so wird das Gas während der gesamten Reaktionszeit eingeleitet. In diesem Fall kann bevorzugt auch in einem Autoklaven umgesetzt werden.

Weiterhin können die Verbindungen der Formel I erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer OH-Gruppe eine OR"-Gruppe tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Tolyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Iodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCI in Dioxan bei 15-60° abgespalten werden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Ferner können die Verbindungen der Formel I hergestellt werden, indem man eine entsprechende Vorstufe einer Verbindung der Formel I durch Reduktion in eine solche überführt. So ist es beispielsweise möglich, eine Verbindung, die an sich einer Verbindung der Formel I entspricht, aber anstelle von R³ über einen Alkinylsubstituenten verfügt, dieses durch partielle katalytische Hydrierung in eine Verbindung der Formel I zu überführen. Derartige Reduktionen werden vorzugsweise mit Wasserstoffgas in Gegenwart eines Katalysators, wie z.B. Pd, Pt, Lindlar-Katalysator etc. hydriert.

Weiterhin kann man eine Verbindung der Formel I in eine andere Verbindung der Formel I überführen, indem man eine Doppelbindung in einem Substituenten R³ isomerisiert. Die Isomerisierung wird zweckmäßig in Gegenwart eines Katalysators in der Hitze durchgeführt. Geeignete Katalysatoren sind z.B. Salze von Pt oder Pd, andere Übergangsmetallkatalysatoren, oder Metallcarbonyle. Besonders bevorzugt ist z.B. Bis(benzonitril)-palladium(ll)-chlorid. Die Methode wird von Durvasula et al. in Tetrahedron Lett. 22, 2337 (1981) beschrieben.

Eine Base der Formel I kann ferner mit einer Säure in das zugehörige Säureadditionssalz übergeführt werden. Für diese Umsetzung kommen Säuren in Frage, die physiologische unbedenkliche Salze liefern. So können anorganische Säuren verwendet werden, z.B. Schwefelsäure. Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure. Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und -disulfonsäuren, Laurylschwefelsäure.

Die Verbindungen der Formel I und ihre physiologisch unbedenklichen Salze könne zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden.

Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Hirnödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankunken, Fibrosen sowie Organhypertrophien und -hyperplasien, insbesondere bei Erkrankungen der Prostata.

Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

Man fügt zu einer Lösung von 1,2 g 2-Methyl-4-(1-cyclopenten-3-yl)-5-methylsulfonyl-benzoesäurechlorid [erhältlich durch Umsetzung von 2-Methyl-4-brom-5-methyl-sulfonyl-benzoesäuremethylester mit Cyclopenten in Gegenwart von Pd-(ll)-acetat und Tri-o-tolylphosphin, Trennung der beiden Isomeren mittels einer Prebar^{R}-Edelstahlkartusche 250-50, gefüllt mit LiChroprep RP-18; Eluent 0,1 m NaH₂PO₄-Puffer/Acetonitril 9:1 mit aufsteigendem Gradienten 1:1, und anschließende Verseifung und Überführung ins Säurechlorid mit SOCl₂] in 20 ml Ethylenglycoldimethylether unter Eiskühlung eine frische Guanidinlösung, hergestellt aus 5,6 g Guanidiniumchlorid und 1,6 g Natrium, in 40 ml Ethylenglycoldimethylether hinzu und rührt zwei Stunden bei Raumtemperatur. Anschließend gießt man die Reaktionsmischung auf Eiswasser, säuert an, wäscht mit Essigsäureethylester und stellt den pH-Wert der Lösung auf 10 ein. Nach üblicher Aufarbeitung erhält man das N-Diaminomethylen-2-methyl-4-(1-cyclopenten-3-yl)-5-methylsulfonyl-benzamid, welches aus Acetonitril/ Diethylether umkristallisiert werden kann, F. 212-214°.

Analog erhält man durch Umsetzung von Guanidin
mit 2-Methyl-4-(1-propen-1-yl)-5-methylsulfonyl-benzoesäurechlorid das N-Diaminomethylen-2-methyl-4-(1-propen-1-yl)-5-methylsulfonylbenzamid, F. 202-204°;
mit 2-Methyl-4-ethenyl-5-methylsulfonyl-benzoesäurechlorid das N-Diaminomethylen-2-methyl-4-ethenyl-5-methylsulfonyl-benzamid;
mit 2-Methyl-4-(1-propen-2-yl)-5-methylsulfonyl-benzoesäurechlorid das N-Diaminomethylen-2-methyl-4-(1-propen-2-yl)-5-methylsulfonylbenzamid, F. 168°;
mit 2-Methyl-4-(1-propan-3-yl)-5-methylsulfonyl-benzoesäurechlorid das N-Diaminomethylen-2-methyl-4-(1-propen-3-yl)-5-methylsulfonylbenzamid;
mit 2-Methyl-4-(1-buten-4-yl)-5-methylsulfonyl-benzoesäurechlorid das N-Diaminomethylen-2-methyl-4-(1-buten-4-yl)-5-methylsulfonylbenzamid;
mit 2-Methyl-4-(2-methyl-1-propen-1-yl)-5-methylsulfonyl-benzoesäurechlorid das
   N-Diaminomethylen-2-methyl-4-(2-methyl-1 -propen-1 -yl)-5-methylsulfonyl-benzamid;
mit 2-Methyl-4-(2-buten-1-yl)-5-methylsulfonyl-benzoesäurechlorid das N-Diaminomethylen-2-methyl-4-(2-buten-1-yl)-5-methylsulfonylbenzamid;
mit 2-Methyl-4-(2-buten-2-yl)-5-methylsulfonyl-benzoesäurechlorid das N-Diaminomethylen-2-methyl-4-(2-buten-2-yl)-5-methylsulfonylbenzamid;
mit 2-Methyl-4-(3-methyl-2-buten-2-yl)-5-methylsulfonylbenzoesäurechlorid das N-Diaminomethylen-2-methyl-4-(3-methyl-2-buten-2-yl)-5-methylsulfonyl-benzamid;
mit 2-Methyl-4-(1-buten-3-yl)-5-methylsulfonyl-benzoesäurechlorid das N-Diaminomethylen-2-methyl-4-(1-buten-3-yl)-5-methylsulfonylbenzamid, F. 186-188°;
mit 2-Methyl-4-(1-buten-1-yl)-5-methylsulfonyl-benzoesäurechlorid das N-Diaminomethylen-2-methyl-4-(1 -buten-1 -yl)-5-methylsulfonylbenzamid;
mit 2-Methyl-4-(1-buten-2-yl)-5-methylsulfonyl-benzoesäurechlorid das N-Diaminomethylen-2-methyl-4-(1-buten-2-yl)-5-methylsulfonylbenzamid;
mit 2-Methyl-4-(1-buten-3-yl)-5-methylsulfonyl-benzoesäurechlorid das N-Diaminomethylen-2-methyl-4-(1-buten-3-yl)-5-methylsulfonylbenzamid;
mit 2-Methyl-4-(1-penten-1-yl)-5-methylsulfonyl-benzoesäurechlorid das N-Diaminomethylen-2-methyl-4-(1-penten-1-yl)-5-methylsulfonylbenzamid;
mit 2-Methyl-4-(2-penten-1-yl)-5-methylsulfonyl-benzoesäurechlorid das N-Diaminomethylen-2-methyl-4-(2-penten-1-yl)-5-methylsulfonylbenzamid;
mit 2-Methyl-4-(3-penten-1-yl)-5-methylsulfonyl-benzoesäurechlorid das N-Diaminomethylen-2-methyl-4-(3-penten-1-yl)-5-methylsulfonylbenzamid;
mit 2-Methyl-4-(1-cyclopenten-4-yl)-5-methylsulfonyl-benzoesäurechlorid das N-Diaminomethylen-2-methyl-4-(1-cyclopenten-4-yl)-5-methylsulfonyl-benzamid;
mit 2-Methyl-4-(1-hexen-1-yl)-5-methylsulfonyl-benzoesäurechlorid das N-Diaminomethylen-2-methyl-4-(1 -hexen-1 -yl)-5-methylsulfonylbenzamid;
mit 2-Methyl-4-(2-hexen-1-yl)-5-methylsulfonyl-benzoesäurechlorid das N-Diaminomethylen-2-methyl-4-(2-hexen-1-yl)-5-methylsulfonylbenzamid;
mit 2-Methyl-4-(3-hexen-1-yl)-5-methylsulfonyl-benzoesäurechlorid das N-Diaminomethylen-2-methyl-4-(3-hexen-1-yl)-5-methylsulfonylbenzamid.

### Beispiel 2

Analog Beispiel 1 erhält man durch Umsetzung von 1,8 g 2-Ethyl-4-(1-cyclopenten-3-yl)-5-methylsulfonyl-benzoesäurechlorid mit Guanidin nach üblicher Aufarbeitung das N-Diaminomethylen-2-ethyl-4-(1-cyclopenten-3-yl)-5-methylsulfonyl-benzamid, welches aus Acetonitril/Diethylether umkristallisiert werden kann.

Analog erhält man durch Umsetzung von Guanidin
mit 2-Ethyl-4-(1-propen-1-yl)-5-methylsulfonyl-benzoesäurechlorid das N-Diaminomethylen-2-ethyl-4-(1-propen-1-yl)-5-methylsulfonylbenzamid;
mit 2-Ethyl-4-ethenyl-5-methylsulfonyl-benzoesäurechlorid das N-Diaminomethylen-2-ethyl-4-ethenyl-5-methylsulfonyl-benzamid;
mit 2-Ethyl-4-(1-propen-2-yl)-5-methylsulfonyl-benzoesäurechlorid das N-Diaminomethylen-2-ethyl-4-(1-propen-2-yl)-5-methylsulfonylbenzamid;
mit 2-Ethyl-4-(1-propen-3-yl)-5-methylsulfonyl-benzoesäurechlorid das N-Diaminomethylen-2-ethyl-4-(1-propen-3-yl)-5-methylsulfonylbenzamid;
mit 2-Ethyl-4-(1-buten-4-yl)-5-methylsulfonyl-benzoesäurechlorid das N-Diaminomethylen-2-ethyl-4-(1-buten-4-yl)-5-methylsulfonylbenzamid;
mit 2-Ethyl-4-(2-ethyl-1-propen-1-yl)-5-methylsulfonyl-benzoesäurechlorid das N-Diaminomethylen-2-ethyl-4-(2-ethyl-1 -propen-1 -yl)-5-methylsulfonyl-benzamid;
mit 2-Ethyl-4-(2-buten-1-yl)-5-methylsulfonyl-benzoesäurechlorid das N-Diaminomethylen-2-ethyl-4-(2-buten-1-yl)-5-methylsulfonylbenzamid;
mit 2-Ethyl-4-(2-buten-2-yl)-5-methylsulfonyl-benzoesäurechlorid das N-Diaminomethylen-2-ethyl-4-(2-buten-2-yl)-5-methylsulfonylbenzamid;
mit 2-Ethyl-4-(3-methyl-2-buten-2-yl)-5-methylsulfonyl-benzoesäurechlorid das
   N-Diaminomethylen-2-ethyl-4-(3-methyl-2-buten-2-yl)-5-methylsulfonyl-benzamid;
mit 2-Ethyl-4-(1-buten-3-yl)-5-methylsulfonyl-benzoesäurechlorid das N-Diaminomethylen-2-ethyl-4-(1-buten-3-yl)-5-methylsulfonylbenzamid;
mit 2-Ethyl-4-(1-buten-1-yl)-5-methylsulfonyl-benzoesäurechlorid das N-Diaminomethylen-2-ethyl-4-(1-buten-1-yl)-5-methylsulfonylbenzamid;
mit 2-Ethyl-4-(1-buten-2-yl)-5-methylsulfonyl-benzoesäurechlorid das N-Diaminomethylen-2-ethyl-4-(1-buten-2-yl)-5-methylsulfonylbenzamid;
mit 2-Ethyl-4-(1-buten-3-yl)-5-methylsulfonyl-benzoesäurechlorid das N-Diaminomethylen-2-ethyl-4-(1-buten-3-yl)-5-methylsulfonylbenzamid;
mit 2-Ethyl-4-(1-penten-1-yl)-5-methylsulfonyl-benzoesäurechlorid das N-Diaminomethylen-2-ethyl-4-(1-penten-1-yl)-5-methylsulfonylbenzamid;
mit 2-Ethyl-4-(2-penten-1-yl)-5-methylsulfonyl-benzoesäurechlorid das N-Diaminomethylen-2-ethyl-4-(2-penten-1-yl)-5-methylsulfonylbenzamid;
mit 2-Ethyl-4-(3-penten-1-yl)-5-methylsulfonyl-benzoesäurechlorid das N-Diaminomethylen-2-ethyl-4-(3-penten-1-yl)-5-methylsulfonylbenzamid;
mit 2-Ethyl-4-(1-hexen-1-yl)-5-methylsulfonyl-benzoesäurechlorid das N-Diaminomethylen-2-ethyl-4-(1-hexen-1-yl)-5-methylsulfonylbenzamid;
mit 2-Ethyl-4-(2-hexen-1-yl)-5-methylsulfonyl-benzoesäurechlorid das N-Diaminomethylen-2-ethyl-4-(2-hexen-1-yl)-5-methylsulfonylbenzamid;
mit 2-Ethyl-4-(3-hexen-1-yl)-5-methylsulfonyl-benzoesäurechlorid das N-Diaminomethylen-2-ethyl-4-(3-hexen-1-yl)-5-methylsulfonylbenzamid.

### Beispiel 3

Zu einer Suspension von 32 g N-Diaminomethylen-2-methyl-4-brom-5-methylsulfonyl-benzamid [erhältlich durch Umsetzung von 2-Methyl-4-brom-5-methylsulfonyl-benzoylchlorid mit Guanidin], 40 ml Triethylamin, 0,4 g Pd-II-acetat, 0,8 g Tri-o-tolylphosphin und 80 ml Dimethylformamid (DMF) fügt man 80 ml Cyclopenten hinzu und erhitzt die Reaktionsmischung acht Stunden auf 100-110°. Anschließend entfernt man das Lösungsmittel, versetzt mit 300 ml Wasser, säuert an, wäscht mehrmals mit Essigsäureethylester und stellt den pH-Wert der Lösung auf 10 ein.

Nach üblicher Aufarbeitung erhält man eine Isomerenmischung von N-Diamino-methylen-2-methyl-4-(1-cyclopenten-3-yl)-5-methylsulfonylbenzamid (88 %) und N-Diaminomethylen-2-methyl-4-(1-cyclopenten-4-yl)-5-methylsulfonyl-benzamid (15 %). Zur Isomerentrennung wird das Gemisch auf eine Prebar^{R}-Edelstahlkartusche 250-50, gefüllt mit LiChroprep RP-18, gegeben und mit 0,1 m NaH₂PO₄-Puffer/Acetonitril im Verhältnis 9:1 mit aufsteigendem Gradienten bis zum Verhältnis 1:1 eluiert.
- Isomer 1:: N-Diaminomethylen-2-methyl-4-(1-cyclopenten-3-yl)-5-methylsulfonyl-benzamid, F. 212-214°;
- Isomer 2:: N-Diaminomethylen-2-methyl-4-(1-cyclopenten-4-yl)-5-methylsulfonyl-benzamid.

### Beispiel 4

700 mg N-Diaminomethylen-2-methyl-4-(1-cyclopenten-3-yl)-5-methylsulfonyl-benzamid [herstellbar nach Bsp. 1; F. 212-214°] werden in 20 ml Aceton gelöst und unter Rühren mit 6,1 ml Methansulfonsäure versetzt. Nach Filtration und Lyophilisation erhält man N-Diaminomethylen-2-methyl-4-(1-cyclopenten-3-yl)-5-methylsulfonyl-benzamid, Methansulfonat, F. 202-204°.

Analog erhält man aus den freien Basen die folgenden Methansulfonate:
N-Diaminomethylen-4-(1-cyclopenten-3-yl)-5-methylsulfonyl-benzamid, Methansulfonat, F. 225°;
N-Diaminomethylen-2-methyl-4-(1-cyclopenten-4-yl)-5-methylsulfonylbenzamid, Methansulfonat, F. 186°;
N-Diaminomethylen-2-methyl-4-(1-cyclopenten-1-yl)-5-methylsulfonylbenzamid, Methansulfonat, F. 218-220°;
N-Diaminomethylen-2-methyl-4-(1-buten-3-yl)-5-methylsulfonyl-benzamid, Methansulfonat, F. 122°;
N-Diaminomethylen-2-methyl-4-(1-propen-1-yl)-5-methylsulfonylbenzamid, Methansulfonat, F. 207-209°;
N-Diaminomethylen-4-(1-propen-1-yl)-5-methylsulfonylbenzamid, Methansulfonat, F. 250-252°;
N-Diaminomethylen-2-methyl-4-(1-propen-2-yl)-5-methylsulfonylbenzamid, Methansulfonat, F. 172°.

### Beispiel 5

Analog Beispiel 1 erhält man durch Umsetzung von 2,2 g 3-Methylsulfonyl-4-(1-cyclopenten-3-yl)-benzoesäurechlorid mit Guanidin nach üblicher Aufarbeitung das N-Diaminomethylen-3-methylsulfonyl-4-(1-cyclopenten-3-yl)-benzamid, welches aus Acetonitril/Diethylether umkristallisiert werden kann, F. 188-190°.

Analog erhält man durch Umsetzung von Guanidin
mit 3-Methylsulfonyl-4-(1-propen-1-yl)-benzoesäurechlorid das N-Diaminomethylen-3-methylsulfonyl-4-(1-propen-1-yl)-benzamid, F. 223-225°;
mit 3-Methylsulfonyl-4-ethenyl-benzoesäurechlorid das N-Diaminomethylen-3-methylsulfonyl-4-ethenyl-benzamid;
mit 3-Methylsulfonyl-4-(1-propen-2-yl)-benzoesäurechlorid das N-Diaminomethylen-3-methylsulfonyl-4-(1-propen-2-yl)-benzamid;
mit 3-Methylsulfonyl-4-(1-propen-3-yl)-benzoesäurechlorid das N-Diaminomethylen-3-methylsulfonyl-4-(1-propen-3-yl)-benzamid;
mit 3-Methylsulfonyl-4-(1-buten-4-yl)-benzoesäurechlorid das N-Diaminomethylen-3-methylsulfonyl-4-(1-buten-4-yl)-benzamid;
mit 3-Methylsulfonyl-4-(3-Methylsulfonyl-1-propen-1-yl)-benzoesäurechlorid das N-Diaminomethylen-3-methylsulfonyl-4-(3-Methylsulfonyl-1-propen-1-yl)-benzamid;
mit 3-Methylsulfonyl-4-(2-buten-1-yl)-benzoesäurechlorid das N-Diaminomethylen-3-methylsulfonyl-4-(2-buten-1-yl)-benzamid;
mit 3-Methylsulfonyl-4-(2-buten-2-yl)-benzoesäurechlorid das N-Diaminomethylen-3-methylsulfonyl-4-(2-buten-2-yl)-benzamid;
mit 3-Methylsulfonyl-4-(3-methyl-2-buten-2-yl)-benzoesäurechlorid das N-Diaminomethylen-3-methylsulfonyl-4-(3-methyl-2-buten-2-yl)benzamid;
mit 3-Methylsulfonyl-4-(1-buten-3-yl)-benzoesäurechlorid das N-Diaminomethylen-3-methylsulfonyl-4-(1-buten-3-yl)-benzamid;
mit 3-Methylsulfonyl-4-(1-buten-1-yl)-benzoesäurechlorid das N-Diaminomethylen-3-methylsulfonyl-4-(1-buten-1-yl)-benzamid;
mit 3-Methylsulfonyl-4-(1-buten-2-yl)-benzoesdurechlorid das N-Diaminomethylen-3-methylsulfonyl-4-(1-buten-2-yl)-benzamid;
mit 3-Methylsulfonyl-4-(1-buten-3-yl)-benzoesäurechlorid das N-Diaminomethylen-3-methylsulfonyl-4-(1-buten-3-yl)-benzamid;
mit 3-Methylsulfonyl-4-(1-penten-1-yl)-benzoesäurechlorid das N-Diaminomethylen-3-methylsulfonyl-4-(1-penten-1-yl)-benzamid;
mit 3-Methylsulfonyl-4-(2-penten-1-yl)-benzoesäurechlorid das N-Diaminomethylen-3-methylsulfonyl-4-(2-penten-1-yl)-benzamid;
mit 3-Methylsulfonyl-4-(3-penten-1-yl)-benzoesäurechlorid das N-Diaminomethylen-3-methylsulfonyl-4-(3-penten-1-yl)-benzamid;
mit 3-Methylsulfonyl-4-(1-hexen-1-yl)-benzoesäurechlorid das N-Diaminomethylen-3-methylsulfonyl-4-(1 -hexen-1 -yl)-benzamid;
mit 3-Methylsulfonyl-4-(2-hexen-1-yl)-benzoesäurechlorid das N-Diaminomethylen-3-methylsulfonyl-4-(2-hexen-1-yl)-benzamid;
mit 3-Methylsulfonyl-4-(3-hexen-1-yl)-benzoesäurechlorid das N-Diaminomethylen-3-methylsulfonyl-4-(3-hexen-1-yl)-benzamid.

### Beispiel 6

6,0 g N-Diaminomethylen-2-methyl-4-ethinyl-5-methylsulfonyl-benzamid, F. 223-226° [erhältlich durch Umsetzung von 2-Methyl-4-brom-5-methylsulfonyl-benzoe-säuremethylester mit Li-acetylid zu 2-Methyl-4-ethinyl-5-methylsulfonyl-benzoesäuremethylester und anschließende Umsetzung mit Guanidin] werden in 300 ml DMF gelöst und in Gegenwart von 450 mg Pd-CaCO₃ (Lindlar-Katalysator) 15 Minuten im Wasserstoffstrom (p = 1 bar) hydriert. Nach üblicher Aufarbeitung erhält man das N-Diaminomethylen-2-methyl-4-ethenyl-5-methylsulfonyl-benzamid.

Analog erhält man durch Hydrierung mit H₂ in Gegenwart des Lindlar-Katalysators
mit N-Diaminomethylen-2-methyl-4-(1-propin-1-yl)-5-methylsulfonylbenzamid das N-Diaminomethylen-2-methyl-4-(1-propen-1-yl)-5-methylsulfonylbenzamid;
mit N-Diaminomethylen-2-methyl-4-(1-propin-3-yl)-5-methylsulfonylbenzamid das N-Diaminomethylen-2-methyl-4-(1-propen-3-yl)-5-methylsulfonylbenzamid;
mit N-Diaminomethylen-2-ethyl-4-ethinyl-5-methylsulfonyl-benzamid das N-Diaminomethylen-2-ethyl-4-ethenyl)-5-methylsulfonyl-benzamid;
mit N-Diaminomethylen-2-ethyl-4-(1-propin-3-yl)-5-methylsulfonylbenzamid das N-Diaminomethylen-2-ethyl-4-(1-propen-3-yl)-5-methylsulfonylbenzamid;
mit N-Diaminomethylen-2-ethyl-4-(1-propin-1-yl)-5-methylsulfonylbenzamid das N-Diaminomethylen-2-ethyl-4-(1 -propen-1 -yl)-5-methylsulfonylbenzamid.

### Beispiel 7

Zu einer Lösung von 1 g N-Diaminomethylen-2-methyl-4-(1-cyclopenten-3-yl)- 5-methylsulfonyl-benzamid [erhältlich nach Bsp. 1] in 20 ml Toluol fügt man 80 mg Bis-(benzonitril)-Pd-(ll)-chlorid hinzu und kocht 2 Stunden unter Rückfluß. Anschließend erhält man nach Filtration, Entfernung des Lösungsmittels und üblicher Aufarbeitung ein Isomerengemisch bestehend aus 3 Isomeren. Nach Vorreinigung des Isomeren-gemisches durch Chromatographie (Kieselgel/Essigsäureethylester - Methanol 9 : 1) wird zur Trennung das Gemisch auf eine Prebar^{R}-Edelstahlkartusche 250-50, gefüllt mit LiChroprep RP-18, gegeben und mit 0,1 m NaH₂PO₄-Puffer/Acetonitril im Verhältnis 9:1 mit aufsteigendem Gradienten bis zum Verhältnis 1:1 eluiert. Man erhält:
- Isomer 1:: N-Diaminomethylen-2-methyl-4-(1-cyclopenten-3-yl)-5-methylsulfonyl-benzamid, F. 184-186° (14 %);
- Isomer 2:: N-Diaminomethylen-2-methyl-4-(1-cyclopenten-1-yl)-5-methylsulfonyl-benzamid, F. 138-140° (42 %);
- Isomer 3:: N-Diaminomethylen-2-methyl-4-(1-cyclopenten-4-yl)-5-methylsulfonyl-benzamid (44 %).

### Beispiel 8

Analog Beispiel 7 erhält man durch Isomerisierung von 3,1 g N-Diaminomethylen-2-ethyl-4-(1-cyclopenten-3-yl)- 5-methylsulfonyl-benzamid [erhältlich nach Bsp. 2] in Gegenwart von Bis-(benzonitril)-Pd-(II)-chlorid:
- Isomer 1:: N-Diaminomethylen-2-ethyl-4-(1-cyclopenten-3-yl)-5-methylsulfonyl-benzamid;
- Isomer 2:: N-Diaminomethylen-2-ethyl-4-(1-cyclopenten-1-yl)-5-methylsulfonyl-benzamid;
- Isomer 3:: N-Diaminomethylen-2-ethyl-4-(1-cyclopenten-4-yl)-5-methylsulfonyl-benzamid.

### Beispiel 9

Analog Beispiel 1 erhält man durch Umsetzung von 1,8 g 2-Methyl-4-(1-cyclopenten-3-yl)-5-nitro-benzoesäurechlorid mit Guanidin nach üblicher Aufarbeitung das N-Diaminomethylen-2-methyl-4-(1-cyclopenten-3-yl)-5-nitro-benzamid, welches aus Acetonitril/Diethylether umkristallisiert werden kann.

Analog erhält man durch Umsetzung von Guanidin
mit 2-Methyl-4-(1-propen-1-yl)-5-nitro-benzoesäurechlorid das N-Diaminomethylen-2-methyl-4-(1-propen-1-yl)-5-nitro-benzamid;
mit 2-Methyl-4-ethenyl-5-nitro-benzoesäurechlorid das N-Diaminomethylen-2-methyl-4-ethenyl-5-nitro-benzamid;
mit 2-Methyl-4-(1-propen-2-yl)-5-nitro-benzoesäurechlorid das N-Diaminomethylen-2-methyl-4-(1-propen-2-yl)-5-nitro-benzamid;
mit 2-Methyl-4-(1-propen-3-yl)-5-nitro-benzoesäurechlorid das N-Diaminomethylen-2-methyl-4-(1-propen-3-yl)-5-nitro-benzamid;
mit 2-Methyl-4-(1-buten-4-yl)-5-nitro-benzoesäurechlorid das N-Diaminomethylen-2-methyl-4-(1-buten-4-yl)-5-nitro-benzamid;
mit 2-Methyl-4-(2-methyl-1-propen-1-yl)-5-nitro-benzoesäurechlorid das N-Diaminomethylen-2-methyl-4-(2-methyl-1-propen-1-yl)-5-nitrobenzamid;
mit 2-Methyl-4-(2-buten-1-yl)-5-nitro-benzoesäurechlorid das N-Diaminomethylen-2-methyl-4-(2-buten-1-yl)-5-nitro-benzamid;
mit 2-Methyl-4-(2-buten-2-yl)-5-nitro-benzoesäurechlorid das N-Diaminomethylen-2-methyl-4-(2-buten-2-yl)-5-nitro-benzamid;
mit 2-Methyl-4-(3-methyl-2-buten-2-yl)-5-nitro-benzoesäurechlorid das N-Diaminomethylen-2-methyl-4-(3-methyl-2-buten-2-yl)-5-nitrobenzamid;
mit 2-Methyl-4-(1-buten-3-yl)-5-nitro-benzoesäurechlorid das N-Diaminomethylen-2-methyl-4-(1-buten-3-yl)-5-nitro-benzamid;
mit 2-Methyl-4-(1-buten-1-yl)-5-nitro-benzoesäurechlorid das N-Diaminomethylen-2-methyl-4-(1-buten-1-yl)-5-nitro-benzamid;
mit 2-Methyl-4-(1-buten-2-yl)-5-nitro-benzoesäurechlorid das N-Diaminomethylen-2-methyl-4-(1-buten-2-yl)-5-nitro-benzamid;
mit 2-Methyl-4-(1-buten-3-yl)-5-nitro-benzoesäurechlorid das N-Diaminomethylen-2-methyl-4-(1-buten-3-yl)-5-nitro-benzamid;
mit 2-Methyl-4-(1-penten-1-yl)-5-nitro-benzoesäurechlorid das N-Diaminomethylen-2-methyl-4-(1-penten-1-yl)-5-nitro-benzamid;
mit 2-Methyl-4-(2-penten-1-yl)-5-nitro-benzoesäurechlorid das N-Diaminomethylen-2-methyl-4-(2-penten-1-yl)-5-nitro-benzamid;
mit 2-Methyl-4-(3-penten-1-yl)-5-nitro-benzoesäurechlorid das N-Diaminomethylen-2-methyl-4-(3-penten-1-yl)-5-nitro-benzamid;
mit 2-Methyl-4-(1-hexen-1-yl)-5-nitro-benzoesäurechlorid das N-Diaminomethylen-2-methyl-4-(1-hexen-1-yl)-5-nitro-benzamid;
mit 2-Methyl-4-(2-hexen-1-yl)-5-nitro-benzoesäurechlorid das N-Diaminomethylen-2-methyl-4-(2-hexen-1-yl)-5-nitro-benzamid;
mit 2-Methyl-4-(3-hexen-1-yl)-5-nitro-benzoesäurechlorid das N-Diaminomethylen-2-methyl-4-(3-hexen-1-yl)-5-nitro-benzamid.

### Beispiel 10

Analog Beispiel 1 erhält man durch Umsetzung von 1,8 g 2-Methyl-4-(1-cyclopenten-3-yl)-5-trifluormethyl-benzoesäurechlorid mit Guanidin nach üblicher Aufarbeitung das N-Diaminomethylen-2-methyl-4-(1-cyclopenten-3-yl)-5-trifluormethyl-benzamid, welches aus Acetonitril/Diethylether umkristallisiert werden kann.

Analog erhält man durch Umsetzung von Guanidin
mit 2-Methyl-4-(1-propen-1-yl)-5-trifluormethyl-benzoesäurechlorid das N-Diaminomethylen-2-methyl-4-(1-propen-1-yl)-5-trifluormethylbenzamid;
mit 2-Methyl-4-ethenyl-5-trifluormethyl-benzoesäurechlorid das N-Diaminomethylen-2-methyl-4-ethenyl-5-trifluormethyl-benzamid;
mit 2-Methyl-4-(1-propen-2-yl)-5-trifluormethyl-benzoesäurechlorid das N-Diaminomethylen-2-methyl-4-(1-propen-2-yl)-5-trifluormethylbenzamid;
mit 2-Methyl-4-(1-propen-3-yl)-5-trifluormethyl-benzoesäurechlorid das N-Diaminomethylen-2-methyl-4-(1-propen-3-yl)-5-trifluormethylbenzamid;
mit 2-Methyl-4-(1-buten-4-yl)-5-trifluormethyl-benzoesäurechlorid das N-Diaminomethylen-2-methyl-4-(1-buten-4-yl)-5-trifluormethylbenzamid;
mit 2-Methyl-4-(2-methyl-1-propen-1-yl)-5-trifluormethyl-benzoesäurechlorid das N-Diaminomethylen-2-methyl-4-(2-methyl-1-propen-1-yl)-5-trifluormethyl-benzamid;
mit 2-Methyl-4-(2-buten-1-yl)-5-trifluormethyl-benzoesäurechlorid das N-Diaminomethylen-2-methyl-4-(2-buten-1-yl)-5-trifluormethylbenzamid;
mit 2-Methyl-4-(2-buten-2-yl)-5-trifluormethyl-benzoesäurechlorid das N-Diaminomethylen-2-methyl-4-(2-buten-2-yl)-5-trifluormethylbenzamid;
mit 2-Methyl-4-(3-methyl-2-buten-2-yl)-5-trifluormethyl-benzoesäurechlorid das N-Diaminomethylen-2-methyl-4-(3-methyl-2-buten-2-yl)-5-trifluormethyl-benzamid;
mit 2-Methyl-4-(1-buten-3-yl)-5-trifluormethyl-benzoesäurechlorid das N-Diaminomethylen-2-methyl-4-(1-buten-3-yl)-5-trifluormethylbenzamid;
mit 2-Methyl-4-(1-buten-1-yl)-5-trifluormethyl-benzoesäurechlorid das N-Diaminomethylen-2-methyl-4-(1-buten-1-yl)-5-trifluormethylbenzamid;
mit 2-Methyl-4-(1-buten-2-yl)-5-trifluormethyl-benzoesäurechlorid das N-Diaminomethylen-2-methyl-4-(1-buten-2-yl)-5-trifluormethylbenzamid;
mit 2-Methyl-4-(1-buten-3-yl)-5-trifluormethyl-benzoesäurechlorid das N-Diaminomethylen-2-methyl-4-(1-buten-3-yl)-5-trifluormethylbenzamid;
mit 2-Methyl-4-(1-penten-1-yl)-5-trifluormethyl-benzoesäurechlorid das N-Diaminomethylen-2-methyl-4-(1-penten-1-yl)-5-trifluormethylbenzamid;
mit 2-Methyl-4-(2-penten-1-yl)-5-trifluormethyl-benzoesäurechlorid das N-Diaminomethylen-2-methyl-4-(2-penten-1-yl)-5-trifluormethylbenzamid;
mit 2-Methyl-4-(3-penten-1-yl)-5-trifluormethyl-benzoesäurechlorid das N-Diaminomethylen-2-methyl-4-(3-penten-1-yl)-5-trifluormethylbenzamid;
mit 2-Methyl-4-(1-hexen-1-yl)-5-trifluormethyl-benzoesäurechlorid das N-Diaminomethylen-2-methyl-4-(1 -hexen-1 -yl)-5-trifluormethylbenzamid;
mit 2-Methyl-4-(2-hexen-1-yl)-5-trifluormethyl-benzoesäurechlorid das N-Diaminomethylen-2-methyl-4-(2-hexen-1-yl)-5-trifluormethylbenzamid;
mit 2-Methyl-4-(3-hexen-1-yl)-5-trifluormethyl-benzoesäurechlorid das N-Diaminomethylen-2-methyl-4-(3-hexen-1-yl)-5-trifluormethylbenzamid.

### Beispiel 11

Analog Beispiel 1 erhält man durch Umsetzung von 1,8 g 2-Methyl-4-(1-cyclohexen-3-yl)-5-methylsulfonyl-benzoesäurechlorid mit Guanidin nach üblicher Aufarbeitung das N-Diaminomethylen-2-methyl-4-(1-cyclohexen-3-yl)-5-methylsulfonyl-benzamid, welches aus Acetonitril/Diethylether umkristallisiert werden kann.

Analog erhält man durch Umsetzung von Guanidin
mit 2-Methyl-4-(1-cyclohexen-3-yl)-5-nitro-benzoesäurechlorid das N-Diaminomethylen-2-methyl-4-(1-cyclohexen-3-yl)-5-nitrobenzamid;
mit 2-Methyl-4-(1-cyclohexen-3-yl)-5-trifluormethyl-benzoesäurechlorid das N-Diaminomethylen-2-methyl-4-(1-cyclohexen-3-yl)-5-trifluormethylbenzamid;
mit 2-Ethyl-4-(1-cyclohexen-3-yl)-5-nitro-benzoesäurechlorid das N-Diaminomethylen-2-ethyl-4-(1-cyclohexen-3-yl)-5-nitro-benzamid.

### Beispiel 12

Analog Beispiel 7 erhält man durch Isomerisierung von 2,3 g N-Diaminomethylen-2-methyl-4-(1-cyclohexen-3-yl)- 5-methylsulfonyl-benzamid [erhältlich nach Bsp. 11] in Gegenwart von Bis-(benzonitril)-Pd-(ll)-chlorid:
- Isomer 1:: N-Diaminomethylen-2-methyl-4-(1-cyclohexen-3-yl)-5-methylsulfonyl-benzamid;
- Isomer 2:: N-Diaminomethylen-2-methyl-4-(1-cyclohexen-1-yl)-5-methylsulfonyl-benzamid;
- Isomer 3:: N-Diaminomethylen-2-methyl-4-(1-cyclohexen-4-yl)-5-methylsulfonyl-benzamid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Alkenyl-benzoylguanidin-Derivate -Derivate der Formel I 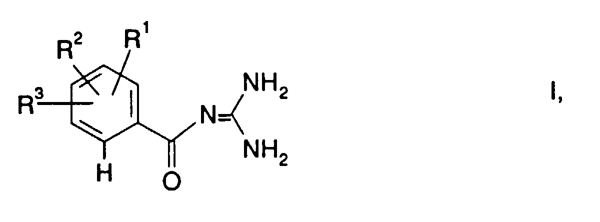 worin
R¹ und R² jeweils unabhängig voneinander H, Hal, A, CN, NO₂, CF₃, CH₂F, CHF₂, C₂F₅, CH₂CF₃ oder SOₙ-R⁴,
R³ -CR⁵=CR⁶R⁷, -C(R⁶R⁵)-CR⁷=CR⁹R⁸, -C(R⁶R⁵)-C(R⁷R⁸)-CR⁹=CR¹⁰R¹¹ oder Cycloalkenyl mit 3-7 C-Atomen oder Cycloalkenylalkyl mit 4-8 C-Atomen,
R⁴ A oder Ph,
R⁵, R⁶, R⁷, R⁸, R⁹, R¹⁰ und R¹¹ jeweils unabhängig voneinander H oder A
A Alkyl mit 1 bis 6 C-Atomen,
Hal F, Cl, Br oder I,
Ph unsubstituiertes oder ein-, zwei- oder dreifach durch A, OA, NR⁴R⁵, F, Cl, Br, I oder CF₃ substituiertes Phenyl und
n 1 oder 2,
bedeuten,
sowie deren physiologisch unbedenkliche Salze.

2. (a) N-Diaminomethylen-2-methyl-4-(1-cyclopenten-3-yl)-5-methylsulfonyl-benzamid;
(b) N-Diaminomethylen-2-methyl-4-(1-propen-1-yl)-5-methylsulfonyl-benzamid;
(c) N-Diaminomethylen-2-methyl-4-ethenyl-5-methylsulfonylbenzamid;
(d) N-Diaminomethylen-2-methyl-4-(1-cyclopenten-1-yl)-5-methylsulfonyl-benzamid;
(e) N-Diaminomethylen-2-methyl-4-(1-propen-3-yl)-5-methylsulfonyl-benzamid;
(f) N-Diaminomethylen-2-methyl-4-(1-cyclohexen-3-yl)-5-methylsulfonyl-benzamid;
gemäß Anspruch 1, sowie deren physiologisch unbedenkliche Salze.

3. Verfahren zur Herstellung von Alkylbenzoylguanidin-Derivaten der Formel I gemäß Anspruch 1 sowie von deren Salzen, dadurch gekennzeichnet, daß man eine Verbindung der Formel II 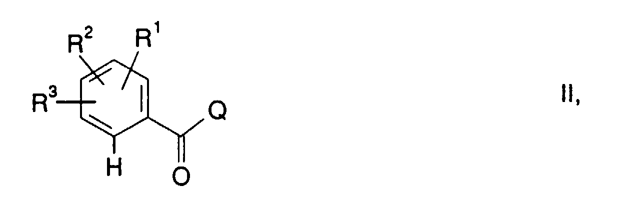 worin R¹, R² und R³ die zuvor angegebenen Bedeutungen haben
und
Q Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
oder daß man ein Benzoylguanidin der Formel III 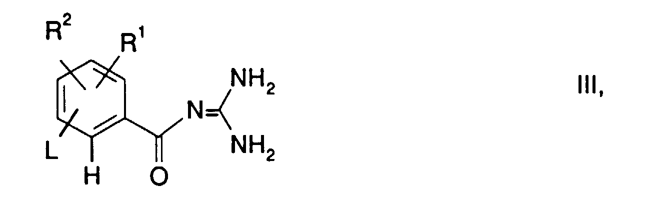 worin R¹ und R² die zuvor angegebenen Bedeutungen besitzen,
und
L F, Cl, Br oder I bedeutet,
mit einer ungesättigten Kohlenwasserstoffverbindung der Formel IV
R³-H (IV),
worin R³ die angegebene Bedeutung besitzt,
in Gegenwart eines Übergangsmetallkatalysators und gegebenenfalls eines Aktivators umsetzt,
oder daß man eine sonst der Formel I entsprechende Verbindung, die jedoch anstelle eines oder mehrerer Wasserstoffatome eine oder mehrere reduzierbare Gruppe(n), wie z.B. eine Alkinylgruppe
und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
oder daß man einen Rest R³ in einen anderen Rest R³ durch Isomerisierung umwandelt, indem man eine Doppelbindung unter Einwirkung eines Übergangsmetallkatalysators und/oder eines Metallcarbonyls umlagert,
und/oder daß man eine erhaltene Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

7. Verwendung von Verbindungen der Formel I nach Anspruch 1 zur Herstellung von Arzneimitteln zur Behandlung von Arrhythmien, Angina pectoris, Infarkten sowie zur präventiven Behandlung der genannten Indikationen.

## Claims

1. Alkenyl-benzoylguanidine derivatives of the formula I 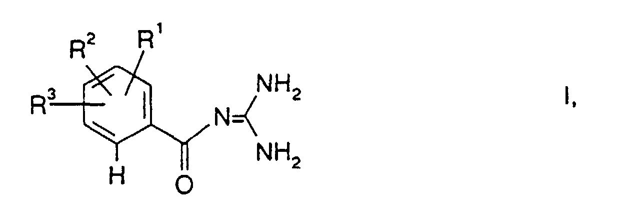 wherein
R¹ and R² in each case independently of one another are H, Hal, A, CN, NO₂, CF₃, CH₂F, CHF₂, C₂F₅, CH₂CF₃ or SOₙ-R₄,
R³ is -CR⁵=CR⁶R⁷, -C(R⁶R⁵ )- CR⁷=CR⁹R⁸, -C(R⁶R⁵)-C(R⁷R⁸)-CR⁹=CR¹⁰R¹¹ or cycloalkenyl having 3-7 C atoms or cycloalkenylalkyl having 4-8 C atoms,
R⁴ is A or Ph,
R⁵, R⁶, R⁷, R⁸, R⁹, R¹⁰, and R¹¹ in each case independently of one another are H or A,
A is alkyl having 1 to 6 C atoms
Hal is F, Cl, Br or I,
Ph is phenyl which is unsubstituted or mono-, di- or trisubstituted by A, OA, NR⁴R⁵, F, Cl, Br, I or CF₃
n is 1 or 2,
and physiologically acceptable salts thereof.

2. (a) N-diaminomethylene-2-methyl-4-(1-cyclopenten-3-yl)-5-methylsulfonylbenzamide;
(b) N-diaminomethylene-2-methyl-4-(1-propen-1-yl)-5-methylsulfonylbenzamide;
(c) N-diaminomethylene-2-ethenyl-5-methylsulfonylbenzamide;
(d) N-diaminomethylene-2-methyl-4-(1-cyclopenten-1-yl)-5-methylsulfonylbenzamide;
(e) N-diaminomethylene-2-methyl-4-(1-propen-3-yl)-5-methylsulfonylbenzamide;
(f) N-diaminomethylene-2-methyl-4-(1-cyclohexen-3-yl)-5-methylsulfonylbenzamide;
according to Claim 1, and physiologically acceptable salts thereof.

3. Process for the preparation of alkylbenzoylguanidine derivatives of the formula I according to Claim 1 and of salts thereof, characterized in that a compound of the formula II 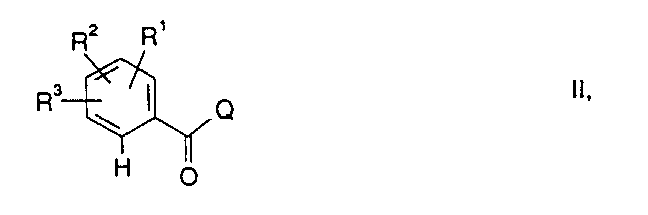 wherein R¹, R² and R³ have the abovementioned meanings
and
Q is Cl, Br, OA, 0-CO-A, O-CO-Ph, OH or another reactive esterified OH group or leaving group which can easily be replaced nucleophilically,
is reacted with guanidine,
or in that a benzoylguanidine of the formula III 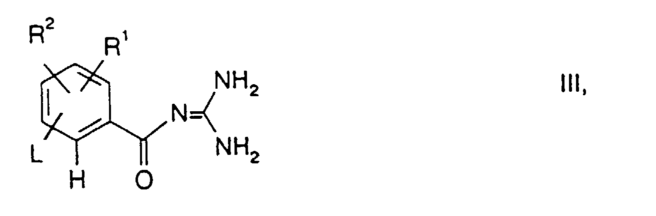 wherein R¹ and R² have the abovementioned meanings
and
L is F, Cl, Br or I,
is reacted with an unsaturated hydrocarbon compound of the formula IV
R³-H IV
wherein R³ has the meaning given,
in the presence of a transition metal catalyst and if appropriate of an activator,
or in that a compound which otherwise corresponds to the formula I but which contains, instead of one or more hydrogen atoms, one or more reducible group(s), such as, for example, an alkynyl group, and/or one or more additional C-C and/or C-N bond(s) is treated with a reducing agent,
or in that a compound which otherwise corresponds to the formula I but which contains, instead of one or more hydrogen atoms, one or more solvolyzable group(s) is treated with a solvolyzing agent,
or in that a radical R³ is converted into another radical R³ by isomerization, by rearranging a double bond under the action of a transition metal catalyst and/or of a metal carbonyl,
and/or in that a base of the formula I obtained is converted into one of its salts by treatment with an acid.

4. Process for the preparation of pharmaceutical formulations, characterized in that a compound of the formula I according to Claim 1 and/or one of its physiologically acceptable salts is brought into a suitable dosage form together with at least one solid, liquid or semi-liquid carrier or auxiliary.

5. Pharmaceutical formulation, characterized in that it comprises at least one compound of the general formula I according to Claim 1 and/or one of its physiologically acceptable salts.

6. Use of compounds of the formula I according to Claim 1 or of physiologically acceptable salts thereof for the preparation of a medicament.

7. Use of compounds of the formula I according to Claim 1 for the preparation of medicaments for treatment of arrhythmias, angina pectoris or infarctions and for preventive treatment of the indications mentioned.

## Revendications

1. Les dérivés d'alcénylbenzoylguanidines de formule I 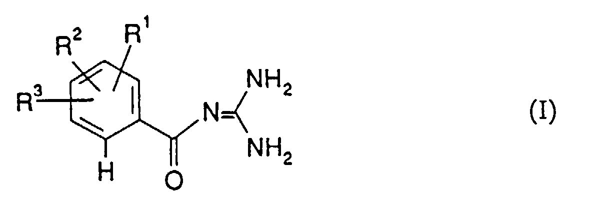 où
R¹ et R² représentent, indépendamment l'un de l'autre, H, Hal, A, CN, NO₂, CF₃, CH₂F, CHF₂, C₂F₅, CH₂CF₃ ou SOₙ -R⁴,
R³ représente -CR⁵ = CR⁶R⁷, -C(R⁶R⁵)-CR⁷ = CR⁹R⁸, -C(R⁶R⁵)-C(R⁷R⁸)-CR⁹ = CR¹⁰R¹¹ ou un cycloalcényle comportant 3 à 7 atomes de C ou un cycloalcénylalkyle comportant 4 à 8 atomes de C,
R⁴ A ou Ph,
R⁵, R⁶, R⁷, R⁸, R⁹, R¹⁰ et R¹¹ représentent, indépendamment les uns des autres, H ou A,
A représente un alkyle comportant 1 à 6 atomes de C,
Hal F, Cl, Br ou I,
Ph un phényle non substitué ou mono-, di- ou trisubstitué par A, OA, NR⁴R⁵, F, Cl, Br, I ou CF₃ et
n est égal à 1 ou 2,
ainsi que leurs sels physiologiquement acceptables.

2. (a) le N-diaminométhylène-2-méthyl-4-(1-cyclopentén-3-yl)-5-méthylsulfonylbenzamide,
(b) le N-diaminométhylène-2-méthyl-4-(1-propén-1-yl)-5-méthylsulfonylbenzamide,
(c) le N-diaminométhylène-2-méthyl-4-éthényl-5-méthylsulfonylbenzamide,
(d) le N-diaminométhylène-2-méthyl-4-(1-cyclopentén-1-yl)-5-méthylsulfonylbenzamide,
(e) le N-diaminométhylène-2-méthyl-4-(1-propén-3-yl)-5-méthylsulfonylbenzamide,
(f) le N-diaminométhylène-2-méthyl-4-(1-cyclohexén-3-yl)-5-méthylsulfonylbenzamide,
selon la revendication 1, ainsi que leurs sels physiologiquement acceptables.

3. Procédé pour la préparation des dérivés d'alkylbenzoylguanidines de formule I selon la revendication 1, ainsi que de leurs sels, caractérisé en ce que l'on fait réagir un composé de formule II : 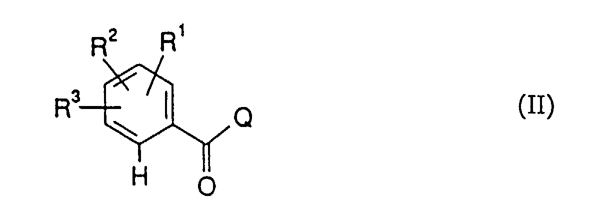 ou R¹, R² et R³ ont les significations précédemment indiquées, et
Q représente Cl, Br, OA, O-CO-A, O-CO-Ph, OH ou un autre groupe OH estérifié réactif ou un groupe partant nucléophile facilement substituable, avec la guanidine,
ou en ce que l'on fait réagir une benzoylguanidine de formule III 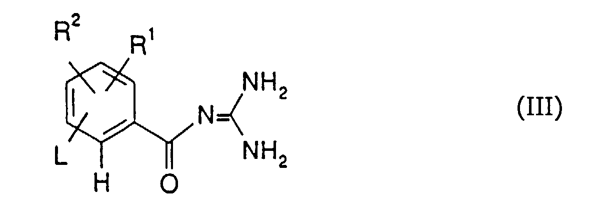 où R¹ et R² ont les significations précédemment indiquées et
L représente F, Cl, Br ou I,
avec un composé hydrocarboné insaturé de formule IV
R³―H (IV)
où R³ a la signification indiquée,
en présence d'un catalyseur à base d'un métal de transition et éventuellement d'un activateur,
ou en ce que l'on traite un composé répondant à la formule I, mais contenant à la place d'un ou plusieurs atome(s) d'hydrogène un ou plusieurs groupe(s) réductible(s) tels que par exemple un groupe alcynyle et/ou une ou plusieurs liaison(s) C-C et/ou C-N supplémentaires, avec un agent réducteur,
ou en ce que l'on traite un composé répondant à la formule I, mais contenant à la place d'un ou plusieurs atome(s) d'hydrogène un ou plusieurs groupe(s) solvolysable(s), avec un agent solvolysant,
ou en ce que l'on transforme un reste R³ en un autre reste R³ par isomérisation, en déplaçant une double liaison sous l'action d'un catalyseur à base d'un métal de transition et/ou d'un carbonyle métallique
et/ou en ce que l'on transforme la base de formule I obtenue en l'un de ses sels par traitement avec un acide.

4. Procédé pour la fabrication de préparations pharmaceutiques, caractérisé en ce que l'on met sous une forme de dosage appropriée un composé de formule I selon la revendication 1 et/ou l'un de ses sels physiologiquement acceptables, associé à au moins un support ou un adjuvant solide, liquide ou semi-liquide.

5. Préparation pharmaceutique caractérisée en ce qu'elle contient au moins un composé de formule générale I selon la revendication 1 et/ou l'un de ses sels physiologiquement acceptables.

6. Utilisation des composés de formule I selon la revendication 1 ou de leurs sels physiologiquement acceptables pour la préparation d'un médicament.

7. Utilisation des composés de formule I selon la revendication 1 pour la préparation de médicaments destinés au traitement de l'arythmie, de l'angine de poitrine, des infarctus, ainsi qu'au traitement préventif des indications précitées.
